# EUROPEAN PATENT SPECIFICATION

(11) **EP 3 414 257 B1**
(45) Date of publication and mention of the grant of the patent: **07.06.2023**
(21) Application number: 17750508.8
(22) Date of filing: 06.02.2017
(51) Int. Cl.: C07K 14/605, C07K 1/10

(54) **METHOD FOR PREPARATION OF LIRAGLUTIDE USING BAL LINKER**
VERFAHREN ZUR HERSTELLUNG VON LIRAGLUTIDE MITHILFE EINES BAL-LINKER
PROCÉDÉ DE PRÉPARATION DE LIRAGLUTIDE À L'AIDE D'UN LIEUR BAL

(30) Priority: 11.02.2016 EP 16155165; 11.02.2016 US 201662293830 P
(43) Date of publication of application: 19.12.2018
(73) Proprietor: Polypeptide Laboratories Holding (PPL) AB, 200 61 Limhamn (SE)
(72) Inventor: CARBAJO LOPEZ, Daniel, 08005 Barcelona (ES); ALBERICIO PALOMERA, Fernando, 08005 Barcelona (ES)
(74) Representative: Rigler, Johann
(86) International application number: PCT/SE2017/000008
(87) International publication number: WO 2017/138855

(56) References cited:
- EP-A1- 2 757 107
- EP-A1- 2 757 107
- WO-A1-2015/013167
- WO-A2-2014/199397
- CN-A- 103 864 918
- JENSEN K J ET AL: "BACKBONE AMIDE LINKER (BAL) STRATEGY FOR SOLID-PHASE SYNTHESIS OF C-TERMINAL-MODIFIED AND CYCLIC PEPTIDES", JOURNAL OF THE AMERICAN CHEMICAL SOCIETY, AMERICAN CHEMICAL SOCIETY, US, vol. 120, 1 January 1998 (1998-01-01), pages 5441-5452, XP001084160, ISSN: 0002-7863, DOI: 10.1021/JA974116F
- GUILLAUMIE , FANNY ET AL.: 'Solid-phase synthesis of C-termianl peptide aldehydes from amino acetals anchored to a backbone amide linker (BAL) handle' TETRAHEDRON LETTERS vol. 41, no. 32, 2000, pages 6131 - 6135, XP004243528

### SEQUENCE LISTING

<110> Lonza Braine S.A.
<120> Method for Preparation of Liraglutide using BAL linker
<130> LP2434EP00
<160> 1
<170> PatentIn version 3.5
<210> 1
   <211> 31
   <212> PRT
   <213> Artificial
<220>
   <223> Synthetic construct
<400> 1
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Arg Gly Arg Gly
               20                  25                  30

## Description

### FIELD OF THE INVENTION

The invention discloses a method for the preparation of liraglutide by SPPS using Backbone Amide Linker (BAL) and on resin introduction during SPPS of Palmitoyl-Glu-OtBu residue on the Lys⁽²⁰⁾.

### BACKGROUND OF THE INVENTION

Liraglutide has the CAS number 204656-20-2 and is compound of formula (3), 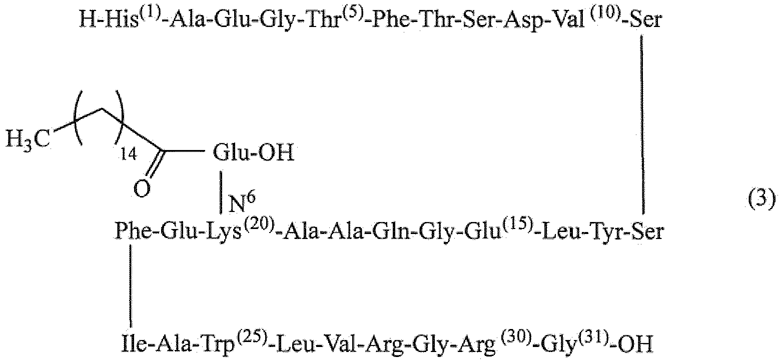 the sequence can also be described as H-His⁽¹⁾-Ala-Glu-Gly-Thr⁽⁵⁾-Phe-Thr-Ser-Asp-Val⁽¹⁰⁾-Ser-Ser-Tyr-Leu-Glu⁽¹⁵⁾-Gly-Gln-Ala-Ala-Lys⁽²⁰⁾(Palmitoyl-Glu)-Glu-Phe-Ile-Ala-Trp⁽²⁵⁾-Leu-Val-Arg-Gly-Arg⁽³⁰⁾-Gly⁽³¹⁾-OH, the numbers in parenthesis showing the numbering of the positions of the AA in the sequence as the numbering is used in instant invention.

WO 98/08871 A1 discloses a method for preparation of derivatives of GLP-1 and analogues thereof by culturing a host cell culture containing a DNA sequence encoding the polypeptide and capable of expressing the polypeptide in a suitable nutrient medium under conditions permitting the expression of the peptide, after which the resulting peptide is recovered from the culture.

WO 00/55119 A1 discloses the acylation of the epsilon-amino residue of Lys⁽²⁰⁾ of GLP-1 analogues with certain compounds useful as acylating agents. In this disclosure the N-terminal amino residue of GLP-1 analogues are not protected. The disadvantage is that diacylation occurs, both the N-terminal amino residue and the epsilon-amino residue of Lys⁽²⁰⁾ are acylated. The undesired by products need to be separated by purification.

EP 2757107 A1 discloses the synthesis of liraglutide by solid phase peptide synthesis (SPPS) on Wang or CTC resin. Neither of the Wang or CTC resins are amine-modified.

WO 2015/013167 A1 discloses several pharmacologically relevant peptides without specifically mention liraglutide. Dependent on the constitution of the C-terminus of the peptide two synthesis schemes are outlined. Peptides with a free carboxylic acid on the C-terminus are synthesized using 2-chlortrityl chloride PS-resin (2-CTC resin). Peptides with an N-monosubstituted carboxamide on the C-terminus are synthesized using a BAL-AM-PS-resin loaded with amines. WO 2015/013167 A1 does not suggest that the use of a BAL -linker over a 2-CTC resin would render an increase in yield or purity.

CN 103864918 A presents solid phase synthesis of liraglutide by the application of a convergent strategy implying the use of three peptide fragments. CN 103864918 A fails to disclose an SPPS scheme incorporating BAL.

Jensen et al., J. Am. Chem. Soc. 1998, 120, 5441-5452, discloses the use of Backbone Amide Linker (BAL) for SPPS of C-terminal-modified peptides.

There was a need for a method for preparation of liraglutide with high yields and that has not the disadvantage of producing by products by said diacylation.

A method was found that uses BAL and incorporates the Palmitoyl-Glu-OtBu residue on the Lys⁽²⁰⁾ during SPPS and on resin while the N-terminal NH₂ is still protected. If it was introduced onto a fully deprotected liraglutide precursor that has not yet the Palmitoyl-Glu-OtBu residue, a method that could be derived from the disclosure of WO 00/55119 A1, then the N-terminal NH₂ first needs to be selectively protected before the Palmitoyl-Glu-OtBu residue can be introduced onto the Lys⁽²⁰⁾ in order to avoid diacylation. This required selective protection is an additional difficulty, an additional source of impurities and introduces further steps in the synthesis of liraglutide.

Another advantage of the method is that the Palmitoyl-Glu-OtBu residue can be introduced with a good yield and with low side reactions by introducing it after the coupling of Ala⁽¹⁸⁾ or after the coupling of Ala⁽¹⁹⁾, thereby no or only low amounts of by products are formed, which would need to be separated. The method provides for high yields and low impurity profiles.

The following abbreviations are used in this specification:
- AA: amino acid;
- Acm: acetamidomethyl;
- ACN: acetonitrile;
- Alloc: allyloxycarbonyl protecting group;
- BAL: Backbone Amide Linker, also called BAL Linker;
- Boc: tert-butoxycarbonyl protecting group;
- coupling: also called coupling reaction;
- DCM: dichloromethane;
- DIC: Diisopropylcarbodiimide;
- DIPCDI: Diisopropylcarbodiimide;
- DMF: Dimethylformamide;
- Dnp: dinitrophenyl;
- eq: equivalents;
- Fmoc: 9-fluorenylmethoxycarbonyl protecting group;
- HFIP: hexafluoroisopropanol;
- MIS: 1,2-dimethylindole-3-sulfonyl;
- Mtr: 4-methoxy-2,3,6-trimethylphenylsulfonyl;
- N⁶: N⁶ denotes the NH₂ of the side chain of Lys;
- N-terminal NH₂: N-terminal amino function;
- OxymaPure^{®}: Ethyl (hydroxyimino)cyanoacetate, CAS 3849-21-6, purchased from Luxembourg Bio Technologies;
- Pal: Palmitoyl;
- Palm: Palmitoyl;
- Pbf: 2,2,4,6,7-pentamethyldihydrobenzofuran-5-sulfonyl protecting group;
- Pmc: 2,5,7,8-pentamethylchroman-6-sufonyl;
- RT: room or ambient temperature;
- SPPS: Solid Phase Peptide Synthesis;
- tBu: tert butyl or tBu protecting group;
- OtBu: tert butyl ester;
- TFA: trifluoroacetic acid;
- TFE: trifluoroethanol;
- TIS: triisopropylsilane;
- Trt: trityl;
- Z: benzyloxycarbonyl.

### SUMMARY OF THE INVENTION

Subject of the invention is a method for the preparation of liraglutide with SPPS using a backbone amide linker BAL for connecting the C-terminal Gly⁽³¹⁾ with the resin during SPPS; BAL is compound of formula (BAL); 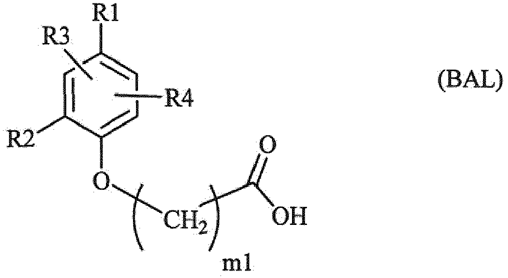 wherein
one of the substituents R1 and R2 is H and the other one is C(O)H;
m1 is 1,2, 3,4, 5, 6, 7, 8, 9 or 10;
R3 and R4 are identical or different and independently from each other H or methoxy, and wherein the SPPS is done on an amino functionalized resin AMFUNRESIN; wherein the Palmitoyl-Glu-OtBu residue on the N⁶ of the Lys⁽²⁰⁾ is the residue of formula (PALGLU) 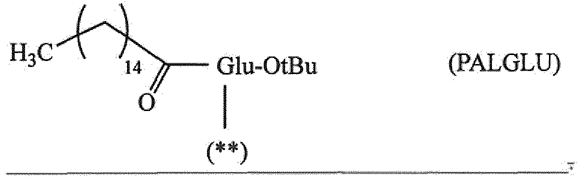
with the (^{∗∗}) in formula (PALGLU) denoting the covalent bond between the COOH of the side chain of the Glu and the N6 of the Lys(20), and the Palmitoyl-Glu-OtBu residue is covalently bonded to the Lys(20) in a reaction REACPALGLU, wherein the NH2 of the side chain of the Lys is reacted with a precursor of the residue of formula (PALGLU); and wherein REACPALGLU is done after the coupling of Ala(19) or after the coupling of Ala(18), and before removal of the protecting group of the alpha NH2 of Ala(19) or of Ala(18) respectively.

### DETAILED DESCRIPTION OF THE INVENTION

Preferably, m1 is 2, 3, 4, 5, 6 or 7; more preferably, m1 is 3, 4, 5 or 7; even more preferably, m1 is 3 or 4; even more preferably, m1 is 3.

Preferably, R3 and R4 are on the 3 and on the 5 position of the phenoxy ring with respect to the position of the carboxylic acid residue on position 1 of the phenoxy ring.

Preferably, R2 is H and R1 is C(O)H.

More preferably, R3 and R4 are on the 3 and on the 5 position of the phenoxy ring with respect to the position of the carboxylic acid residue on position 1 of the phenoxy ring; and R2 is H and R1 is C(O)H.

Even more preferably, R3 and R4 are on the 3 and on the 5 position of the phenoxy ring with respect to the position of the carboxylic acid residue on position 1 of the phenoxy ring; R2 is H and R1 is C(O)H; m1 is 3.

Especially, BAL is compound of formula (BAL-1) which has CAS 197304-21-5.

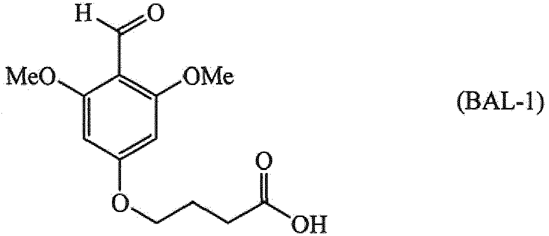

The SPPS is done on an amino functionalized resin AMFUNRESIN.

In principle AMFUNRESIN can be any amino functionalized resin conventionally used in SPPS, since like in whatever SPPS, the structure of the resin and its characteristics (such as substitution, granulometry, percentage of reticulation, and so on) of the resin will influence only the swelling of the resin, but will have no direct implication on the SPPS.

Preferably, AMFUNRESIN is selected from the group consisting of aminomethyl ChemMatrix^{®} resin, aminomethyl polystyrene resin, Rink amide resin, Sieber resin, 4-methyl-benzhydrylamine resin (MBHA resin), poly Lys resin, and benzhydrylamine resin (BHA resin).

Preferably, AMFUNRESIN is an aminomethyl ChemMatrix^{®} resin, Rink amide resin or Sieber resin.

Preferably, the resin used in the SPPS shows a loading capacity of from 0.5 to 2 mmol of reactive sites per g resin.

Preferably, BAL is covalently connected with the resin by an amide bond between the COOH of BAL and the NH₂ of AMFUNRESIN.

Preferably, Gly⁽³¹⁾ is covalently connected with BAL by a bond between the alpha NH₂ of Gly⁽³¹⁾ and the C atom of the C(O)H residue of BAL.

Preferably, Gly⁽³¹⁾ is used with its alpha COOH in protected form, preferably in the form of H-Gly-OtBu, that is with an tBu ester on the alpha COOH.

Preferably, liraglutide is prepared by SPPS by firstly covalently connecting the BAL with AMFUNRESIN. Thereafter the alpha NH₂ of Gly⁽³¹⁾ is covalently connected with the C(O)H residue of BAL. Then the other AA are coupled by SPPS onto the Gly⁽³¹⁾ and then onto the growing peptide chain respectively.

The covalently connecting of BAL with AMFUNRESIN is done in a reaction REACBONBALRES; REACBONBALRES is a coupling reaction and can be done with the reagents and the conditions as described for COUPL.

Preferably, the molar amount of BAL used in REACBONBALRES is from 1 to 10 times, more preferably from 1 to 8 times, even more preferably from 2 to 7 times, of the molar loading capacity of the resin.

Preferably, the covalently connecting of the alpha NH₂ of Gly⁽³¹⁾ with the C(O)H residue of BAL is done with a reductive amination reaction, which comprises two consecutive reactions, a reaction REACONBALGLY1, which is a condensation reaction, and a reaction REACBALGLY2, which is a reduction.

Preferably, REACONBALGLY1 and REACBALGLY2 are done in a solvent.

Preferably, REACONBALGLY1 is done in the presence of an acid, the acid is preferably AcOH.

Preferably, the volume of acid is from 50 to 250 micorL per g of resin, more preferably from 75 to 150 microL per g of resin.

Preferably, the acid is used in a mixture of from 0.1 to 10%, preferably from 0.2 to 5%, more preferably from 0.2 to 3%, of acid with the solvent, the % are vol-% and are being based on the total volume of the mixture of acid and solvent.

REACBALGLY2 is preferably done with a reducing agent, the reducing agent is preferably NaBH₃CN.

Preferably, the molar amount of reducing agent is from 1 to 20 times, more preferably from 1 to 15 times, of the molar loading capacity of the resin.

Preferably, the reducing agent is used in a mixture of from 0.1 to 1 0%, preferably from 0.2 to 5%, more preferably from 0.2 to 3%, of reducing agent with a solvent, the % are vol-% and are being based on the total volume of the mixture of reducing agent and solvent.

The solvent for the two reactions and for the reducing agent can be any solvent that is inert against the reducing agent used in the reduction, preferably, the solvent is DMF, MeOH, THF, MeTHF, NMP or a mixture thereof; more preferably DMF, MeOH or a mixture thereof.

Preferably, REACONBALGLY1 and REACBALGLY2 are done at atmospheric pressure.

Preferably, the reaction temperature of REACONBALGLY1 and of REACBALGLY2 respectively is from -20 to 70°C; more preferably from -15 and 40°C, even more preferably from -15 to 35°C.

Preferably, the reaction time of REACONBALGLY1 and of REACBALGLY2 respectively is from 10 min to 48 h, more preferably from 10 min to 24 h, even more preferably from 10 min to 12 h.

Preferably, the method comprises two steps, a step STEP1 and a step STEP2; STEP1 comprises the SPPS using a backbone amide linker BAL; STEP1 provides liraglutide connected to the resin of the SPPS; STEP2 comprises cleavage of liraglutide from the resin.

Preferably, those AA that can be side chain protected are used in a side chain protected form in the SPPS.

Preferably, STEP1 provides a protected liraglutide connected to the resin of the SPPS.

Preferably, STEP2 comprises deprotection of the protected liraglutide.

Preferably, STEP2 provides liraglutide.

Preferably, the side chain protection is realized in form of side chain protection groups which are not cleavable under weakly acidic conditions, with the weakly acidic conditions as described herein.

Preferably, side chain protection of Cys is Trt or Acm; of Asn and Gln is Trt; of His is Trt or Dnp; of Glu and Asp is OtBu; of Thr, Ser and Tyr is tBu; of Trp is Boc. of Arg is Pbf, Pmc, Mtr or MIS; of Lys is Alloc, Z or Boc.

Preferably, the side chain protection is realized in form of side chain protection groups which are cleavable under strongly acidic conditions, with the strongly acidic conditions as described herein; more preferably except for Lys, where the side chain protection in Alloc, Z or Boc.

More preferably, side chain protection of Cys, Asn, His and Gln is Trt; side chain protection of Glu and Asp is OtBu; side chain protection of Thr, Ser and Tyr is tBu; side chain protection of Trp is Boc; side chain protection of Arg is Pbf; side chain protection of Lys is Alloc.

Preferably, the SPPS is done by stepwise coupling of the individual amino acids, more preferably except for the Val⁽¹⁰⁾ and the Ser⁽¹¹⁾; the Val⁽¹⁰⁾ and the Ser⁽¹¹⁾ are preferably used in the SPPS in form of pseudoproline Fmoc-Val-Ser[Psi^{(Me,Me)}Pro]-OH.

Preferably, the SPPS uses Fmoc/tBu strategy; with Fmoc as protecting group for the alpha NH₂ of the amino acids used in the SPPS; more preferably except for the N-terminal His⁽¹⁾, where the alpha NH₂ is protected with Boc.

Cleavage of the peptide from the resin, of any side chain protecting group, of any protecting group on the N-terminal NH₂, of any protecting group on the C-terminal COOH, also the cleavage of the tBu protecting group on the alpha COOH of the Glu of the Palmitoyl-Glu-OtBu residue, can be done separately or simultaneously. The cleavage is preferably done in STEP2. When the cleavage is done simultaneously, then the cleavage is usually called global deprotection.

The conditions of such cleavage reactions are known to the skilled person and are dependent on the nature of protecting group. Many protecting groups require acidic conditions for cleavage, preferably strongly acidic conditions, with the strongly acidic conditions as defined herein, other protecting groups such as Z require cleavage by catalytic hydrogenation.

Such cleavage reaction or such cleavage reactions can be and is preferably done under strongly acidic conditions, more preferably the cleavage is done in form of a global deprotection.

Strongly acidic conditions means, in the context if this invention, using for cleavage a mixture of from 50 to 100%, preferably from 60 to 100%, more preferably from 70 to 100%, even more preferably from 80 to 100%, of TFA with a component COMPSTRONG, the % are vol-% and are being based on the total volume of the mixture of TFA and COMPSTRONG. Therefore, TFA can also be used neat.

Preferably, COMPSTRONG is selected from the group consisting of SOLVSTRONG, phenol, water, TIS and a mixture thereof; SOLVSTRONG is solvent that is inert against TFA.

Preferably, SOLVSTRONG is DCM; more preferably, COMPSTRONG is a mixture of phenol and TIS or a mixture of phenol, water and TIS.

Preferably, the volume ratios of a mixture TFA: water : TIS are TFA: from 80 to 98; water: from 1 to 10; TIS: from 1 to 10; with the individual volume ratios of the three components adding up to 100.

Preferably, the volume ratios of a mixture TFA: phenol: water : TIS are TFA: from 70 to 97; phenol: from 1 to 10; water: from 1 to 10; TIS: from 1 to 10; with the individual volume ratios of the four components adding up to 100.

Preferably, the total amount of the mixture is from 5 to 20 ml per g of protected peptide. Preferably, the cleavage is done at atmospheric pressure.

Preferably, the reaction temperature of the cleavage is from -20 to 70°C; more preferably from -15 and 40°C, even more preferably from -15 to 35°C.

Preferably, the reaction time of the cleavage is from 30 min to 12 h, more preferably from 30 min to 6 h, even more preferably from 30 min to 4 h.

Weakly acidic conditions means, in the context if this invention, using for cleavage a mixture of from 0.01 to 25%, preferably from 0.01 to 15%, more preferably from 0.05 to 10%, even more preferably from 0.1 to 7.5%, TFA in a solvent SOLVWEAK, the % are vol-% and are being based on the total volume of the mixture of TFA and SOLVWEAK. SOLVWEAK is any solvent which is inert against TFA.

Preferably SOLVWEAK is DCM, methyl THF or a mixture thereof.

Preferably, the total amount of the mixture is from 5 to 20 ml per g of protected peptide. Preferably, the cleavage is done at atmospheric pressure.

Preferably, the reaction temperature of the cleavage is from -20 to 70°C; more preferably from -15 and 40°C, even more preferably from -15 to 35°C.

Preferably, the reaction time of the cleavage is from 5 min to 12 h, more preferably from 5 min to 6 h, even more preferably from 10 min to 4 h.

Cleavage under weakly acidic conditions can also be done using TFE or HFIP instead of TFA.

The Palmitoyl-Glu-OtBu residue on the N⁶ of the Lys⁽²⁰⁾ is the residue of formula (PALGLU), 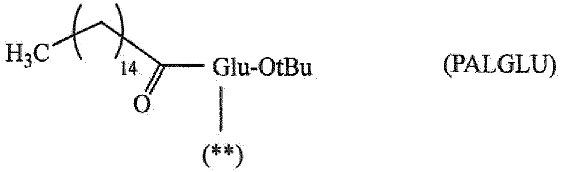 with the (**) in formula (PALGLU) denoting the covalent bond between the COOH of the side chain of the Glu and the N⁶ of the Lys⁽²⁰⁾, and the Palmitoyl-Glu-OtBu residue is covalently bonded to the Lys⁽²⁰⁾ in a reaction REACPALGLU, wherein the NH₂ of the side chain of the Lys is reacted with a precursor of the residue of formula (PALGLU); preferably, REACPALGLU is done before the peptide is cleaved from the resin. So REACPALGLU is done on-resin, that is, REACPALGLU is done while the peptide is still covalently bonded to the resin.

Preferably, the precursor of the residue of formula (PALGLU) is Pal-Glu(OSu)-OtBu, that is, a compound of formula (10); 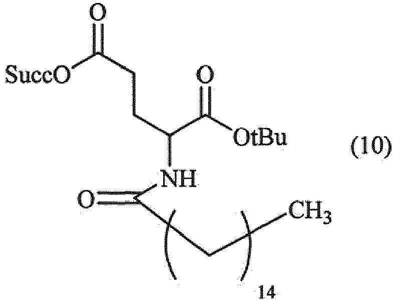 with SuccO being residue of formula (SuccO); 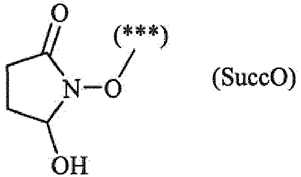 wherein the (***) denotes the covalent bond to the CO residue in compound of formula (10).

REACPALGLU is done after the coupling of Ala⁽¹⁹⁾ or after the coupling of Ala⁽¹⁸⁾, and before removal of the protecting group of the alpha NH₂ of Ala⁽¹⁹⁾ or of Ala⁽¹⁸⁾ respectively, preferably after the coupling of Ala⁽¹⁸⁾.

Preferably, any protecting group of the side chain of Lys⁽²⁰⁾ is cleaved before REACPALGLU.

Preferably, Lys⁽²⁰⁾ is used in the SPPS in form of Fmoc-Lys(Alloc)-OH; so the side chain protecting group of Lys⁽²⁰⁾, which is cleaved before REACPALGLU, is preferably Alloc.

Preferably, cleavage of Alloc from the Lys⁽²⁰⁾ is done with a reaction CLEAVALLOC using Pd(PPh₃)₄ and PhSiH. CLEAVALLOC can be done in the presence of an additive ADD ALLOC, ADDALLOC is selected from the group consisting of morpholine, 1,3-dimethylbarbituric acid, pyrrolidine, triphenylphosphine, dimedone, and mixtures thereof.

Preferably, the molar amount of ADDALLOC is from 2 to 7 times of the molar amount of the Alloc residue.

Preferably, the molar amount of Pd(PPh₃)₄ is from 0.01 to 10 times, more preferably from 0.02 to 5 times, even more preferably from 0.05 to 2 times, of the molar loading capacity of the resin.

Preferably, the molar amount of PhSiH is from 1 to 30 times, more preferably from 2 to 20 times, even more preferably from 5 to 15 times, of the molar loading capacity of the resin.

Preferably, CLEAVALLOC is done in a solvent selected from the group consisting of DMSO, dichloromethane, Me-THF, DMF, NMP, and mixtures thereof, more preferably the solvent is DCM.

Preferably, CLEAVALLOC is done at atmospheric pressure.

Preferably, the reaction temperature of CLEAVALLOC is from -20 to 70°C; more preferably from -15 and 60°C, even more preferably from 5 to 35°C.

Preferably, the reaction time of CLEAVALLOC is from 1 min to 2 h.

Preferably, the molar amount of Pal-Glu(OSu)-OtBu in REACPALGLU is from 1 to 15 times, more preferably from 1 to 10 times, even more preferably from 1 to 5 times, of the molar loading capacity of the resin.

REACPALGLU can be done in the presence of a base, preferably a tertiary amine base. Suitable bases are for example tri alkyl amines, like N,N-diisopropylethylamine (DIPEA) or triethylamine (TEA); N,N-dialkylanilines, like N,N-diethylaniline; 2,4,6-trialkylpyridines, like 2,4,6-trimethylpyridine; and N-alkylmorpholines, like N-methylmorpholine.

Preferably, REACPALGLU is done in the presence of DIPEA as a base.

Preferably, the molar amount of base is from 1 to 10 times, more preferably from 3 to 8 times, of the molar loading capacity of the resin.

Preferably, REACPALGLU is done in a solvent selected from the group consisting of DMSO, dichloromethane, Me-THF, DMF, NMP, and mixtures thereof, more preferably the solvent is DCM.

Preferably, REACPALGLU is done at atmospheric pressure.

Preferably, the reaction temperature of REACPALGLU is from -20 to 70°C; more preferably from -15 and 60°C, even more preferably from 5 to 35°C.

Preferably, the reaction time of REACPALGLU is from 1 to 48 h, more preferably from 5 h to 24 h, even more preferably from 10 h to 24 h.

Preferably, any coupling, which for the ease of reading is also called COUPL, such as coupling during SPPS or REACBONBALRES, can be carried out using reaction conditions known in the art of peptide synthesis.

Preferably, coupling reagents, which can be used for COUPL and which are used in situ, are for example phoshonium or uronium coupling reagents, like benzotriazol-1-yloxy-tris(dimethylamino)phosphonium hexafluorophosphate (BOP), benzotriazol-1-yloxy-tris(pyrrolidino )phosphonium hexafluorophosphate (PyBOP), O-(benzotriazol-1-yl)-1,1,3,3-tetramethyluronium hexafluorophosphate (HBTU), O-(6-chlorobenzotriazol-1-yl)-1,1,3,3-tetramethyluronium hexafluorophosphate (HCTU), O-(6-chlorobenzotriazol-1-yl)-1,1,3,3-tetramethyluronium tetrafluoroborate (TCTU), O-(7-azabenzotriazol-1-yl)-1,1,3,3-tetramethyluronium hexafluorophosphate (HATU), O-(7-azabenzotriazol-1-yl)-1,1,3,3-tetramethyluronium tetrafluoroborate (TATU), O-(benzotriazol-1-yl)-1,1,3,3-tetramethyluronium tetrafluoroborate (TBTU), O-[cyano(ethoxycarbonyl)methylenamino]-1,1,3,3-tetramethyluronium tetrafluoroborate (TOTU) and (1-cyano-2-ethoxy-2-oxoethylidenaminooxy)dimethylamino-morpholino-carbenium hexafluorophosphate (COMU); or carbodiimide coupling reagents, like diisopropylcarbodiimide (DIC), dicyclohexylcarbodiimide (DCC) and water-soluble carbodiimides (WSCDI) like 1-ethyl-3-(3-dimethylaminopropyl)carbodiimide (EDC), optionally as salt like as hydrochloride salt.

Other coupling techniques use pre-formed active esters, such as N-hydroxysuccinimide (HOSu) and p-nitrophenol (HONp) esters, pre-formed symmetrical anhydrides, non-symmetrical anhydrides such as N-carboxyanhydrides (NCAs) and acid halides, such as acyl fluorides or acyl chlorides.

Preferred coupling reagents are carbodiimide coupling reagents and phosphonium coupling reagents, most preferred coupling reagents are selected from group consisting of PyBOP, DCC, DIC and EDC; more preferably, coupling reagent is PyBOP or DIC.

EDC is preferably used as a salt, more preferably as EDC·HCl.

Preferably, the molar amount of coupling reagent is from 1 to 10 times, more preferably from 1 to 5 times, of the molar loading capacity of the resin, or of the molar amount of the fragment to be coupled in LPPS respectively.

More preferably, the molar amount of coupling reagent is from 1 to 10 times, more preferably from 1 to 5 times, of the molar amount of the substrate, such as the AA, to be coupled.

COUPL can be done in the presence of a base, preferably a tertiary amine base, which both deprotonates the COOH residue of the carboxylic component and neutralizes any counterion of the NH₂ residue of the amino component in COUPL, and thus facilitates the coupling reaction.

Suitable bases are for example trialkylamines, like N,N-diisopropylethylamine (DIPEA) or triethylamine (TEA); N,N-dialkylanilines, like N,N-diethylaniline; 2,4,6-trialkylpyridines, like 2,4,6-trimethylpyridine; N,N-dialkylaminopyridines, like N,N-4-dimethylaminopyridine; and N-alkylmorpholines, like N-methylmorpholine.

Preferably, COUPL is done in the presence of DIPEA as a base.

Preferably, the molar amount of base is from 0.01 to 20 times, more preferably from 0.02 to 10 times, of the molar loading capacity of the resin. More preferably, the molar amount of base is from 0.01 to 20 times, even more preferably from 0.02 to 10 times, of the molar amount of the substrate, such as the AA, to be coupled.

COUPL can be done in the presence of an auxiliary nucleophile as additive due to their positive effect in suppressing undesired side reactions. Any known auxiliary nucleophile may be used.

Examples of suitable auxiliary nucleophiles are ethyl (hydroxyimino)cyanoacetate, 1-hydroxybenzotriazole (HOBt), N-hydroxysuccinimide (HOSu), N-hydroxy-3,4-dihydro-4-oxo-1,2,3-benzotriazine (HOOBt) and 1-hydroxy-7-azabenzotriazole (HOAt).

Preferably, HOBt or HOAt are used as auxiliary nucleophile.

Preferably, the molar amount of auxiliary nucleophile is from 0.5 to 8 times of the molar amount of the amino residue that is to be coupled.

Especially, COUPL is done using DIC/OxymaPure, DCC/HOBt, EDC/HOBt, PyBOP/HOBt or EDC/HOAt.

Preferably, COUPL can be done in any inert solvent which can dissolve the reactants.

Preferred solvents for COUPL are water-miscible solvents like dimethyl sulfoxide (DMSO), dioxane, tetrahydrofurans such as tetrahydrofurane (THF) or methyltetrahydrofurane (Methyl-THF), 1-methyl-2-pyrrolidone (NMP), N,N-dimethylformamide (DMF), N,N-dimethylacetamide (DMA), or any mixture thereof; and non water-miscible solvents like dichloromethane (DCM), ethyl acetate or any mixture thereof; and any suitable mixture between water-miscible and non water-miscible solvents, including mixtures with water.

More preferably, COUPL is done in a solvent selected from the group consisting of DMSO, dichloromethane, Me-THF, DMF, NMP, and mixtures thereof.

Preferably, the substrate in COUPL, for example any AA, that is reacted with the respective functional residue connected to the resin in SPPS, is preferably used in excess with regard to the molar loading capacity of the resin; more preferably, it is used in an molar amount of from 1 to 10 times, more preferably from 1 to 8 times, based on the molar loading capacity of the resin.

Preferably, COUPL is done at atmospheric pressure.

Preferably, the reaction temperature of COUPL is from -20 to 70°C; more preferably from -15 and 40°C, even more preferably from -15 to 35°C.

Preferably, the reaction time of COUPL is from 30 min to 48 h, more preferably from 30 min to 24 h, even more preferably from 30 min to 12 h, especially from 30 min to 6 h.

Preferably, the progress of COUPL is monitored by HPLC and thereby the necessary reaction time is determined.

Fmoc cleavage is known to the skilled person, it is done with a base, preferably the base is a secondary amine, more preferably piperidine. The base is used in excess with regard to the molar loading capacity of the resin.

Preferably, DMF is used as solvent in the Fmoc cleavage reaction.

Preferably, the Fmoc cleavage is done at atmospheric pressure.

Preferably, the reaction temperature of the Fmoc cleavage is from -20 to 70°C; more preferably from -15 and 40°C, even more preferably from -15 to 35°C.

Preferably, the reaction time of the Fmoc cleavage is from 1 min to 12 h, more preferably from 1 min to 5 h, even more preferably from 1 min to 1 h.

Liraglutide can be isolated, preferably after a global deprotection, according to standard methods known to the skilled such as precipitation, preferably with ether, centrifugation, filtration etc.

Liraglutide can be purified according to standard methods known to the skilled person, such as HPLC.

### DESCRIPTION OF PREFERRED EMBODIMENTS

*Amino acids.* The SPPS was done with the following AA, if not stated otherwise: Boc-His(Trt)-OH; Fmoc-Ala-OH; Fmoc-Glu(OtBu)-OH; Fmoc-Gly-OH; Fmoc-Thr(tBu)-OH; Fmoc-Phe-OH; Fmoc-Ser(tBu)-OH; Fmoc-Asp(OtBu)-OH; Frnoc-Tpr(tBu)-OH; Fmoc-Val-Ser[Psi(Me,Me)Pro]-OH optionally for position (10) and (11); Fmoc-Leu-OH; Fmoc-Gln(Trt)-OH; Fmoc-Lys(Alloc)-OH; Fmoc-Ile-OH; Fmoc-Trp(Boc)-OH; Fmoc-Val-OH; Fmoc-Arg(Pbf)-OH. SPPS was done manually without an automatic synthesizer.

*Palm-Glu(OSu)-OtBu,* that is compound of formula (10), was purchased from IRIS Biotech; 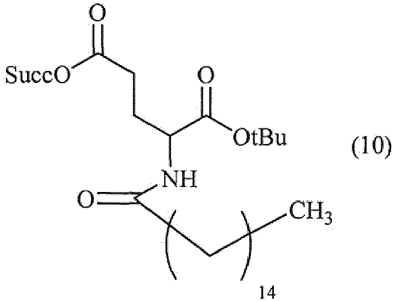 with SuccO being residue of formula (SuccO); 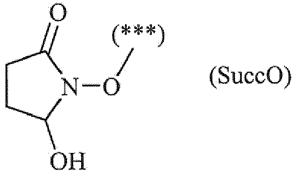 wherein the (^{∗∗∗}) denotes the covalent bond to the CO residue in compound of formula (10). Compound of formula (BAL-1) was purchased from Novabiochem.

*Ninhydrin Test, also called Kaiser Test.* The test is known to the skilled person, see Kaiser E. et al., Analytical Biochemistry 1970, 34, 595-598. Solution 1: Dissolve 5 g ninhydrin in 100 mL ethanol. Solution 2: Dissolve 40 g phenol in 10 mL ethanol. Solution 3: Add 2 mL of a 0.001 M aq. KCN solution to 98 mL pyridine. Procedure: 1. Wash resin with DCM (2 times). 2. Sample a few beads of resin in an Eppendorf tube. 3. Add one to two drops of each of the three solutions 1 to 3. 4. Mix well and heat to 120 °C for 3 to 5 min. 5. The presences of free resin-bound amines are indicated by blue resin beads.

*Aldehyde Test.* Jesus Vazquez et al., Tetrahedron Letters 2001, 42(38), 6691-6693

*Washing with a solvent* / *treating the resin with reagent.* In the following example washing with a solvent such as DMF or DCM was done be filtering the resin, suspending and stirring the resin in the solvent for a certain time and filtering again. All washings were carried out with 10 mL (ca. 15 mL/g of resin) of DMF until Ala⁽¹⁸⁾, then with 15 mL. Stated in the examples are the number of repetitions of this washing cycle together with the time for stirring. Also in case of treating the resin with a reagent that is dissolved in a solvent this description of the number of repetitions of this treatment together with the time for stirring can be used in the example, if not otherwise stated. The introduction of the amino acids was done with 5 mL of DMF, if not otherwise stated.

*Solvent for coupling.* Solvent for coupling was used in the amount of 20 ml/g, if not otherwise stated.

*Yield.* The yield was calculated on the basis of the synthesis scale with respect to resin loading capacity f and resin weight, if not otherwise stated.

*Method for determination of purity.* Peptide-resin (100 mg) is treated with ca. 2 ml of TFA: H₂O : TIS in the volume ratio 94:3:3 and stirring for 1 h at RT. The suspension is filtered. The deprotected peptide is precipitated by addition of diisopropyl ether (20 ml) to the filtrate. The purity of crude deprotected peptide is checked by HPLC: Determination of purity of crude liraglutide: Column with C18 stationary phase; mobile phase A: 0.1% (v/v) TFA in water; mobile phase B: 0.1% (v/v) TFA in acetonitrile; gradient: 5% (v/v) B to 100% (v/v) B in 11 min; temperature: RT; flow rate : 1 ml/min; detection: 254 nm

*Fmoc determination, also used for determination of resin loading.* An analytical method based on the UV absorption of dibenzofulvene after cleavage of Fmoc on an aliquot of peptide-resin, the method is known to the skilled person. It can be done as follows. An aliquot of loaded resin (ca. 100 mg) is washed with DMF (3 times with 5 ml) and DCM (3 times with 5 ml). It is then dried under vacuum.10 mg of dried resin is added to a 2 mL polypropylene tube, 0.8 mL of DMF is added and the resin is allowed to swell for 10 minutes. Then 0.2 mL of piperidine is added, then the mixture is shaken for 20 min. Then 10 microL of the supernatant is pipetted into a quartz cuvette and is diluted with 0.990 mL of DMF. The absorbance at lamda = 301 nm is determined. The piperidine-dibenzofulvene adduct that is formed upon Fmoc deprotection has a molar extinction coefficient of epsilon = 7800 L × mol-1 × cm-1; thus, the loading value is determined using Beer-Lambert law.

### EXAMPLE 1

*Synthesis of Liraglutide according to the invention.* Synthesis of Liraglutide on ChemMatrix^{®} resin was done according to Scheme 1:

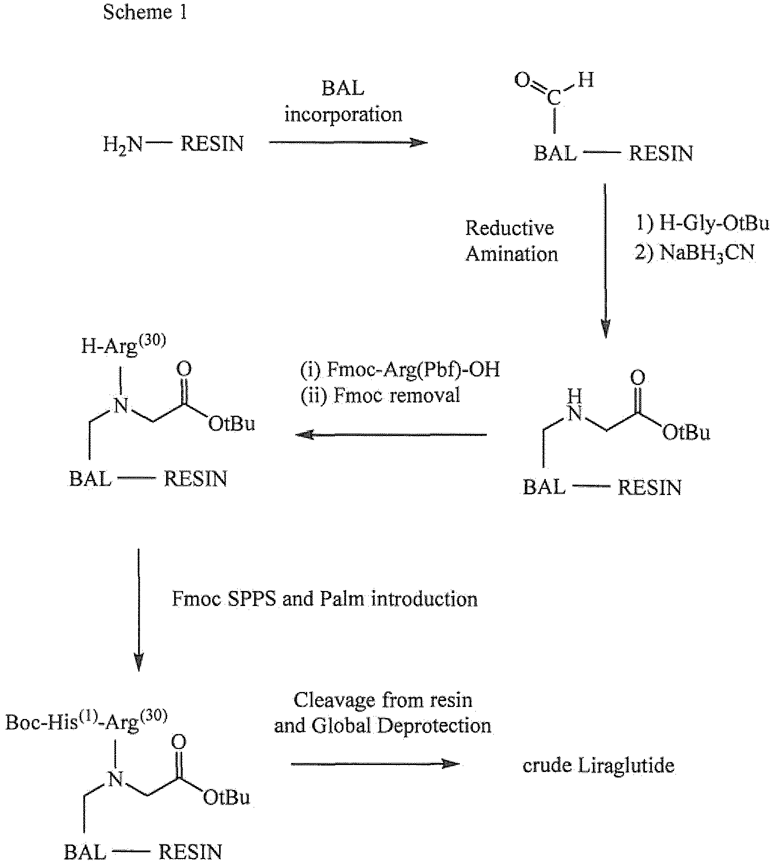

*BAL incorporation.* The synthesis was performed using Aminomethyl ChemMatrix^{®} (purchased from ChemMatrix^{®} Innovation) resin (f = 0.58 mmol/g). The resin (0.6 g, 1 eq) was washed with DCM (3 times). After that, BAL linker (6 eq, 560 mg) was coupled onto the resin with DIC/OxymaPure^{®} (6 eq, 263 microL/296 mg) in DMF. The resin was then stirred for 4 hours. Then the resin was filtered and was washed with DMF (5 times for 1 min each) and DCM (5 times for 1 min each). Ninhydrin test was performed (negative) and aldehyde test (positive) confirmed that the BAL linker was effectively coupled.

*Reductive Amination.* The resin was suspended in DMF and introduction of H-Gly-OtBu was carried out by on-resin reductive amination using the free amine H-Gly-OtBu (10 eq, 579 mg) in form of a solution in 1% (v/v) AcOH in DMF (ca. 100 microL of AcOH per g of resin) for 30 min and then adding a solution of NaBH₃CN (10 eq, 218 mg) in 11 ml of MeOH overnight. After that, the resin was washed with DMF (5 times for 1 min each) and DCM (5 times for 1 min each). The reaction was monitored by aldehyde test (negative).

*Arg acylation.* Fmoc-Arg(Pbf)-OH (3 eq, 677mg) was then coupled using DIC/OxymaPure^{®} (3 eq 131 microL/148 mg) in 45 ml of DMF as coupling reagents for 90 min. To avoid amino acid deletion, the coupling of the Fmoc-Arg(Pbf)-OH was repeated under the same conditions. After that, Fmoc group was removed [by (i) washing the resin with DMF (5 times for 1 min each); (ii) treatment of the resin in piperidine : DMF (2 : 8 v/v, 5 ml/g resin) (1 time for 1 min and 2 times for 10 min each); (iii) washing with DMF (5 times for 1 min each)]. Ninhydrin test was performed with a positive result.

*Coupling of the remaining AA.* Coupling of the remaining AA was carried out by adding the reagents AA : DIC : OxymaPure^{®} in the ratio 3 eq :3 eq :3 eq and then stirring for 90 min. The amino acids Val⁽¹⁰⁾ and Ser⁽¹¹⁾ were coupled in form of the pseudo-Proline Fmoc-Val-Ser(psi^{Me,Me}pro)-OH.

*Removal of Fmoc.* Removal of Fmoc was done by treatment with piperidine : DMF (2 : 8 v/v, 5 ml/g resin) for 1 time for 1 min and 1 time for 15 min.

*Palm introduction.* During the synthesis, Lys was introduced as Fmoc-Lys(Alloc)-OH. After the coupling of Ala⁽¹⁸⁾ and before the removal of its Fmoc protecting group, the Alloc protecting group of the side chain of Lys⁽²⁰⁾ was removed with Pd(PPh₃)₄/PhSiH [by (i) washing the resin with DCM (5 times for 1 min); (ii) treatment of the resin at RT with Pd(PPh₃)₄/PhSiH in the ratio 0.1 eq : 10 eq (2 times for 10 min in DCM); (iii) washing with DCM (5 times for 1 min)]. Then, the resin was treated with Palm-Glu(OSu)-OtBu (3 eq) and DIPEA (6 eq, 265 microL) in DCM for 16 h. After Palm introduction the coupling was continued.

*Cleavage from resin & Global Deprotection & Isolation of crude liraglutide.* After peptide backbone was finished, the peptidyl-resin was washed with DCM (5 time for 1min) and MeOH (5 times for 1 min). Cleavages were done in batches of 0.2 to 0.5 g portions. The cleavage was done with 10 mL of a cocktail [TFA : H₂O : TIS (94 : 3 : 3 volume)]/g of resin. After filtration the resin was washed twice with TFA : H₂O : TIS (94 : 3 : 3 volume) (5 mL) and the TFA solution was concentrated to one third of its volume and then added onto tert-butylmethyl ether (5 times of the volume of the cleavage solution after concentration) and centrifuged. After decantation, the solid was washed twice with the same amount of tert-butylmethyl ether and again centrifuged and decanted. The solid was lyophilized. Yield of crude was 1.06 g, purity of crude was 62 %.

*Purification.* The following purification method is a standard purification method which is not optimized for liraglutide and for yield. The crude unprotected Liraglutide was purified on XSelect^{®} CSH C18 semi-preparative HPLC column (Waters). Mobile phase A: 0.1% (v/v) TFA in water. Mobile phase B: 0.1% TFA (v/v) in acetonitrile. Gradient:

| **t** | **A** | **B** |
|---|---|---|
| **[min]** | **[%]** | **[%]** |
| **0** | 95 | 5 |
| **3** | 95 | 5 |
| **4** | 51 | 49 |
| **9** | 48.5 | 51.5 |
| **10** | 0 | 100 |
| **12** | 0 | 100 |
| **12.5** | 95 | 5 |

Liraglutide elutes at about minute 7 of the gradient. Purity: >99%.Purification yield: ca. 10% .

### EXAMPLE 2

Example 1 was repeated with the difference that the amino acids Val⁽¹⁰⁾ and Ser⁽¹¹⁾ were coupled in form of Fmoc-Ser(tBu)-OH and of Fmoc-Val-OH respectively, that is they were not used on form of a pseudo-Proline. Purity of crude liraglutide obtained without using pseudo-Proline was ca. 40%.

### EXAMPLE 3 (not according to invention)

Example 1 was repeated with the difference that the introduction of the Palmitoyl-Glu-OtBu residue was not done after Ala(18), but after the final coupling of Boc-His(Trt)-OH using the same procedure. This means that after the final coupling of Boc-His(Trt)-OH the Alloc on Lys⁽²⁰⁾ side chain was the selectively cleaved and then the Palmitoyl-Glu-OtBu residue was introduced on resin. Purity of crude liraglutide was ca. 60%

## Claims

1. Method for the preparation of liraglutide with SPPS using a backbone amide linker BAL for connecting the C-terminal Gly⁽³¹⁾ with the resin during SPPS; wherein BAL is a compound of formula (BAL); 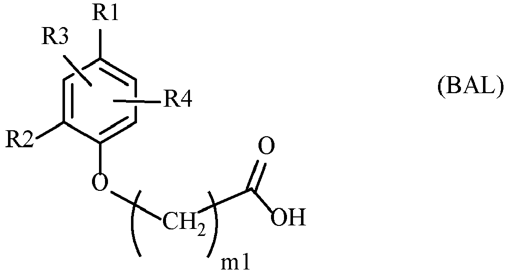 wherein
one of the substituents R1 and R2 is H and the other one is C(O)H;
m1 is 1, 2, 3, 4, 5, 6, 7, 8, 9 or 10;
R3 and R4 are identical or different and independently from each other H or methoxy; and wherein the SPPS is done on an amino functionalized resin AMFUNRESIN; wherein the Palmitoyl-Glu-OtBu residue on the N⁶ of the Lys⁽²⁰⁾ is the residue of formula (PALGLU) 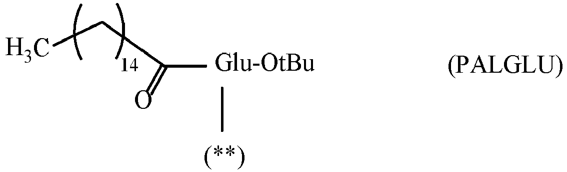
with the (**) in formula (PALGLU) denoting the covalent bond between the COOH of the side chain of the Glu and the N⁶ of the Lys⁽²⁰⁾, and the Palmitoyl-Glu-OtBu residue is covalently bonded to the Lys⁽²⁰⁾ in a reaction REACPALGLU, wherein the NH₂ of the side chain of the Lys is reacted with a precursor of the residue of formula (PALGLU); and wherein REACPALGLU is done after the coupling of Ala⁽¹⁹⁾ or after the coupling of Ala⁽¹⁸⁾, and before removal of the protecting group of the alpha NH₂ of Ala⁽¹⁹⁾ or of Ala⁽¹⁸⁾ respectively.

2. The method according to claim 1, wherein m1 is 2, 3, 4, 5, 6 or 7.

3. The method according to claim 1 or 2, wherein R3 and R4 are on the 3 and on the 5 position of the phenoxy ring with respect to the position of the carboxylic acid residue onposition 1 of the phenoxy ring.

4. The method according to one or more of claims 1 to 3, wherein R2 is H and R1 is C(O)H.

5. The method according to one or more of claims 1 to 4, wherein AMFUNRESIN is selected from the group consisting of aminomethyl ChemMatrix resin, aminomethyl polystyrene resin, Rink amide resin, Sieber resin, 4-methyl-benzhydrylamine resin (MBHA resin), poly Lys resin, and benzhydrylamine resin (BHA resin).

6. The method according to one or more of claims 1 to 5, wherein BAL is covalently connected with the resin by an amide bond between the COOH of BAL and the NH₂ of AMFUNRESIN.

7. The method according to one or more of claims 1 to 6, wherein Gly⁽³¹⁾ is covalently connected with BAL by a bond between the alpha NH₂ of Gly⁽³¹⁾ and the C atom of the C(O)H residue of BAL.

8. The method according to one or more of claims 1 to 7, wherein liraglutide is prepared by SPPS by firstly covalently connecting the BAL with AMFUNRESIN, thereafter the alpha NH₂ of Gly⁽³¹⁾ is covalently connected with the C(O)H residue of BAL, then the other AA are coupled by SPPS onto the Gly⁽³¹⁾ and then onto the growing peptide chain respectively.

9. The method according to claim 7 or 8, wherein the covalently connecting of the alpha NH₂ of Gly⁽³¹⁾ with the C(O)H residue of BAL is done with a reductive amination reaction

10. The method according to one or more of claims 1 to 9, wherein the method comprises two steps, a step STEP1 and a step STEP2; STEP1 comprises the SPPS using a backbone amide linker BAL; STEP1 provides liraglutide connected to the resin of the SPPS; STEP2 comprises cleavage of liraglutide from the resin.

11. The method according to one or more of claims 1 to 10, wherein those AA, that can be side chain protected, are used in a side chain protected form in the SPPS.

12. The method according to claim 11, wherein the side chain protection is realized in form of side chain protection groups which are not cleavable under weakly acidic conditions.

13. The method according to claim 11 or 12, wherein side chain protection of Cys is Trt or Acm; side chain protection of Asn and Gln is Trt; side chain protection of His is Trt or Dnp; side chain protection of Glu and Asp is OtBu; side chain protection of Thr, Ser and Tyr is tBu; side chain protection of Trp is Boc; side chain protection of Arg is Pbf, Pmc, Mtr or MIS; side chain protection of Lys is Alloc, Z or Boc.

14. The method according to one or more of claims 1 to 13, wherein Val⁽¹⁰⁾ and Ser⁽¹¹⁾ are used in the SPPS in form of pseudoproline Fmoc-Val-Ser[Psi^{(Me,Me)}Pro]-OH.

15. The method according to one or more of claims 1 to 14, wherein SPPS uses Fmoc/tBu strategy.

16. The method according to one or more of claims 1 to 15, wherein REACPALGLU is done before the peptide is cleaved from the resin.

17. The method according to one or more of claims 1 to 16, wherein the precursor of the residue of formula (PALGLU) is a compound of formula (10); 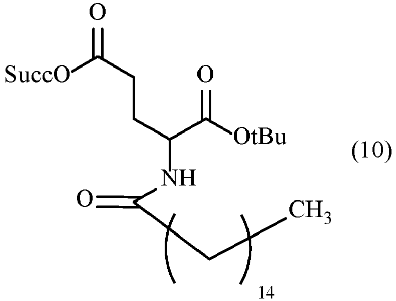 with SuccO being residue of formula (SuccO); 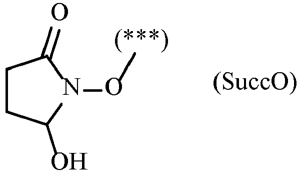 wherein the (***) denotes the covalent bond to the CO residue in compound of formula (10).

## Patentansprüche

1. Verfahren zur Herstellung von Liraglutid mittels SPPS unter Verwendung eines Amidlinker-Rückgrats BAL zum Verbinden des C-terminalen Gly⁽³¹⁾ mit dem Harz während SPPS; wobei BAL eine Verbindung der Formel (BAL) ist; 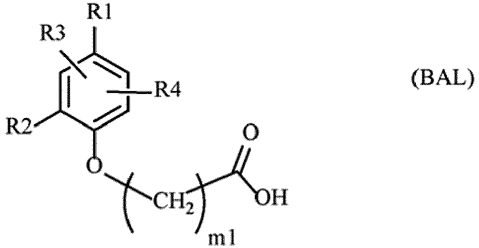 wobei
einer der Substituenten R1 und R2 H ist und der andere C(O)H ist;
m1 1, 2, 3, 4, 5, 6, 7, 8, 9 oder 10 ist;
R3 und R4 gleich oder verschieden und unabhängig voneinander H oder Methoxy sind; und wobei die SPPS auf einem aminofunktionalisierten AMFUNRESIN-Harz durchgeführt wird; wobei der Palmitoyl-Glu-OtBu-Rest am N⁶ des Lys⁽²⁰⁾ der Rest der Formel (PALGLU) ist 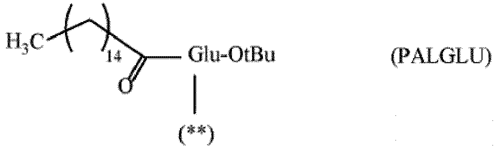
mit (**) in Formel (PALGLU) bezeichnend die kovalente Bindung zwischen dem COOH der Seitenkette des Glu und dem N⁶ des Lys⁽²⁰⁾, und der Palmitoyl-Glu-OtBu-Rest kovalent an das Lys⁽²⁰⁾ in einer REACPALGLU-Reaktion gebunden wird, wobei das NH₂ der Seitenkette des Lys mit einem Vorläufer des Restes der Formel (PALGLU) umgesetzt wird; und wobei REACPALGLU nach der Kupplung von Ala⁽¹⁹⁾ oder nach der Kupplung von Ala⁽¹⁸⁾, und vor einer Entfernung der Schutzgruppe des alpha-NH₂ von Ala⁽¹⁹⁾ beziehungsweise von Ala⁽¹⁸⁾, durchgeführt wird.

2. Verfahren nach Anspruch 1, wobei m1 2, 3, 4, 5, 6 oder 7 ist.

3. Verfahren nach Anspruch 1 oder 2, wobei sich R3 und R4 in der 3- und der 5-Position des Phenoxyrings in Bezug auf die Position des Carbonsäurerests auf Position 1 des Phenoxyrings befinden.

4. Verfahren nach einem oder mehreren der Ansprüche 1 bis 3, wobei R2 H ist und R1 C(O)H ist.

5. Verfahren nach einem oder mehreren der Ansprüche 1 bis 4, wobei AMFUNRESIN ausgewählt ist aus der Gruppe bestehend aus Aminomethyl-ChemMatrix-Harz, Aminomethyl-Polystyrol-Harz, Rink-Amid-Harz, Sieber-Harz, 4-Methyl-Benzhydrylamin-Harz (MBHA-Harz), Poly-Lys-Harz und Benzhydrylamin-Harz (BHA-Harz).

6. Verfahren nach einem oder mehreren der Ansprüche 1 bis 5, wobei BAL durch eine Amidbindung zwischen dem COOH von BAL und dem NH₂ von AMFUNRESIN kovalent mit dem Harz verbunden ist.

7. Verfahren nach einem oder mehreren der Ansprüche 1 bis 6, wobei Gly⁽³¹⁾ kovalent mit BAL durch eine Bindung zwischen dem alpha-NH₂ von Gly⁽³¹⁾ und dem C-Atom des C(O)H-Rests von BAL verbunden ist.

8. Verfahren nach einem oder mehreren der Ansprüche 1 bis 7, wobei Liraglutid durch SPPS hergestellt wird, indem zunächst das BAL mit AMFUNRESIN kovalent verbunden wird, danach das alpha-NH₂ von Gly⁽³¹⁾ kovalent mit dem C(O)H-Rest von BAL verbunden wird, dann die anderen AA durch SPPS an das Gly⁽³¹⁾ und dann respektive an die wachsende Peptidkette gekoppelt werden.

9. Verfahren nach Anspruch 7 oder 8, wobei das kovalente Verbinden des alpha-NH₂ von Gly⁽³¹⁾ mit dem C(O)H-Rest von BAL durch eine reduktive Aminierungs-Reaktion durchgeführt wird.

10. Verfahren nach einem oder mehreren der Ansprüche 1 bis 9, wobei das Verfahren zwei Schritte umfasst, einen Schritt STEP1 und einen Schritt STEP2; STEP1 umfasst die SPPS unter Verwendung eines Amidlinker-Rückgrats BAL; STEP1 liefert Liraglutid, das mit dem Harz der SPPS verbunden ist; STEP2 umfasst ein Abspalten von Liraglutid vom Harz.

11. Verfahren nach einem oder mehreren der Ansprüche 1 bis 10, wobei diejenigen AA, die Seitenketten-geschützt sein können, in einer Seitenketten-geschützten Form in der SPPS verwendet werden.

12. Verfahren nach Anspruch 11, wobei der Seitenkettenschutz in Form von Seitenkettenschutzgruppen, die unter schwach sauren Bedingungen nicht spaltbar sind, realisiert wird.

13. Verfahren nach Anspruch 11 oder 12, wobei Seitenkettenschutz von Cys Trt oder Acm ist; Seitenkettenschutz von Asn und Gin Trt ist; Seitenkettenschutz von His Trt oder Dnp ist; Seitenkettenschutz von Glu und Asp OtBu ist; Seitenkettenschutz von Thr, Ser und Tyr tBu ist; Seitenkettenschutz von Trp Boc ist; Seitenkettenschutz von Arg Pbf, Pmc, Mtr oder MIS ist; Seitenkettenschutz von Lys Alloc, Z oder Boc ist.

14. Verfahren nach einem oder mehreren der Ansprüche 1 bis 13, wobei Val⁽¹⁰⁾ und Ser⁽¹¹⁾ in der SPPS in Form von Pseudoprolin Fmoc-Val-Ser[Psi^{(Me,Me)}Pro]-OH verwendet werden.

15. Verfahren nach einem oder mehreren der Ansprüche 1 bis 14, wobei SPPS Fmoc/tBu-Strategie verwendet.

16. Verfahren nach einem oder mehreren der Ansprüche 1 bis 15, wobei REACPALGLU durchgeführt wird, bevor das Peptid vom Harz abgespalten wird.

17. Verfahren nach einem oder mehreren der Ansprüche 1 bis 16, wobei der Vorläufer des Restes der Formel (PALGLU) eine Verbindung der Formel (10) ist; 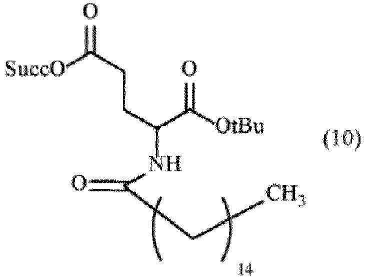 wobei SuccO ein Rest der Formel (SuccO) ist; 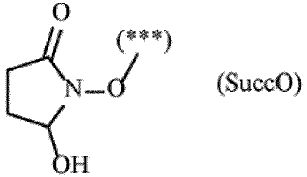 wobei (***) die kovalente Bindung am CO-Rest in der Verbindung der Formel (10) bezeichnet.

## Revendications

1. Procédé pour la préparation de liraglutide avec une SPPS en utilisant un lieur à squelette amide BAL pour relier le Gly⁽³¹⁾ C-terminal avec la résine pendant la SPPS ; où BAL est un composé de formule (BAL) ; 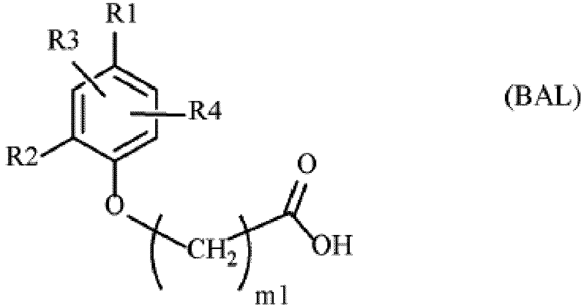 où
un des substituants R1 et R2 est H et l'autre est C(O)H ;
m1 est égal à 1, 2, 3, 4, 5, 6, 7, 8, 9 ou 10 ;
R3 et R4 sont identiques ou différents et indépendamment l'un de l'autre H ou méthoxy ;
et où la SPPS est réalisée sur une résine amino fonctionnalisée AMFUNRESIN ;
où le résidu Palmitoyl-Glu-OtBu sur le N⁶ du Lys⁽²⁰⁾ est le résidu de formule (PALGLU) 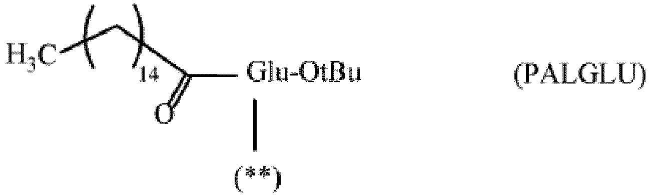
avec le (**) dans la formule (PALGLU) indiquant la liaison covalente entre le COOH de la chaîne latérale du Glu et le N⁶ du Lys⁽²⁰⁾, et le résidu Palmitoyl-Glu-OtBu est lié par covalence au Lys⁽²⁰⁾ dans une réaction REACPALGLU, où le NH₂ de la chaîne latérale du Lys réagit avec un précurseur du résidu de formule (PALGLU) ; et où REACPALGLU est réalisée après le couplage de Ala⁽¹⁹⁾ ou après le couplage de Ala⁽¹⁸⁾, et avant l'élimination du groupe protecteur de l'alpha NH₂ de Ala⁽¹⁹⁾ ou de Ala⁽¹⁸⁾ respectivement.

2. Procédé selon la revendication 1, où m1 est égal à 2, 3, 4, 5, 6 ou 7.

3. Procédé selon la revendication 1 ou 2, où R3 et R4 sont sur la position 3 et sur la position 5 du noyau phénoxy par rapport à la position du résidu acide carboxylique sur la position 1 du noyau phénoy.

4. Procédé selon l'une quelconque des revendications 1 à 3, où R2 est H et R1 est C(O)H.

5. Procédé selon l'une ou plusieurs des revendications 1 à 4, où AMFUNRESIN est choisie dans le groupe consistant en une résine ChemMatrix d'aminométhyle, résine d'aminométhyl polystyrène, résine d'amide Rink, résine Sieber, résine de 4-méthyl-benzhydrylamine (résine MBHA), résine poly Lys, et résine de benzhydrylamine (résine BHA).

6. Procédé selon l'une ou plusieurs des revendications 1 à 5, où BAL est relié par covalence avec la résine par une liaison amide entre le COOH de BAL et le NH₂ de AMFUNRESIN.

7. Procédé selon l'une ou plusieurs des revendications 1 à 6, où Gly⁽³¹⁾ est relié par covalence avec BAL par une liaison entre l'alpha NH₂ de Gly⁽³¹⁾ et l'atome C du résidu C(O)H de BAL.

8. Procédé selon l'une ou plusieurs des revendications 1 à 7, où le liraglutide est préparé par SPPS en reliant dans un premier temps par covalence le BAL avec AMFUNRESIN, après cela l'alpha NH₂ de Gly⁽³¹⁾ est relié par covalence avec le résidu C(O)H de BAL, puis les autres AA sont couplés par SPPS sur le Gly⁽³¹⁾ et puis sur la chaîne de peptide en croissance respectivement.

9. Procédé selon la revendication 7 ou 8, où la liaison covalente de l'alpha NH₂ de Gly⁽³¹⁾ avec le résidu C(O)H de BAL est réalisée avec une réaction d'amination réductrice.

10. Procédé selon l'une ou plusieurs des revendications 1 à 9, où le procédé comprend deux étapes, une étape STEP1 et une étape STEP2 ; STEP1 comprend la SPPS en utilisant un lieur à squelette amide BAL ; STEP1 fournit du liraglutide relié à la résine du SPPS ; STEP2 comprend le clivage de liraglutide à partir de la résine.

11. Procédé selon l'une ou plusieurs des revendications 1 à 10, où les AA, qui peuvent être protégés en chaîne latérale, sont utilisés dans une forme protégée en chaîne latérale dans la SPPS.

12. Procédé selon la revendication 11, où la protection de chaîne latérale est réalisée dans la forme de groupes de protection de chaîne latérale qui ne peuvent pas être clivés dans des conditions faiblement acides.

13. Procédé selon la revendication 11 ou 12, où la protection de chaîne latérale de Cys est Trt ou Acm ; la protection de chaîne latérale de Asn et Gln est Trt ; la protection de chaîne latérale de His est Trt ou Dnp ; la protection de chaîne latérale de Glu et Asp est OtBu ; la protection de chaîne latérale de Thr, Ser et Tyr est tBu ; la protection de chaîne latérale de Trp est Boc ; la protection de chaîne latérale de Arg est Pbf, Pmc, Mtr ou MIS ; la protection de chaîne latérale de Lys est Alloc, Z ou Boc.

14. Procédé selon l'une ou plusieurs des revendications 1 à 13, où Val⁽¹⁰⁾ et Ser⁽¹¹⁾ sont utilisés dans la SPPS dans la forme de pseudoproline Fmoc-Val-Ser[Psi^{(Me,Me)}Pro]-OH.

15. Procédé selon l'une ou plusieurs des revendications 1 à 14, où SPPS utilise une stratégie Fmoc/tBu.

16. Procédé selon l'une ou plusieurs des revendications 1 à 15, où REACPALGLU est réalisée avant que le peptide soit clivé de la résine.

17. Procédé selon l'une ou plusieurs des revendications 1 à 16, où le précurseur du résidu de formule (PALGLU) est un composé de formule (10) ; 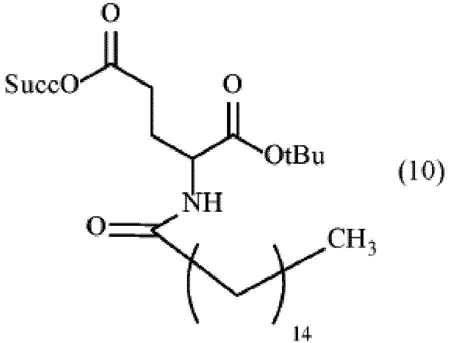 avec SuccO étant un résidu de formule (SuccO) ; 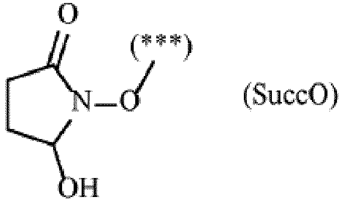 où le (***) indique la liaison covalente au résidu CO dans le composé de formule (10).
